# EUROPEAN PATENT SPECIFICATION

(11) **EP 2 563 163 B1**
(45) Date of publication and mention of the grant of the patent: **22.10.2014**
(21) Application number: 11710503.1
(22) Date of filing: 28.03.2011
(51) Int. Cl.: A23L 1/03, A23L 1/24, A23L 1/22, C12N 9/24, C12N 9/42, C12N 9/88, A23L 1/212, A23D 7/005

(54) **EDIBLE PRODUCT AND USE OF SUCH PRODUCT FOR INCREASING THE BIOAVAILABILITY OF MICRONUTRIENTS COMPRISED IN VEGETABLES OR FRUIT**
ESSBARES PRODUKT UND VERWENDUNG ZUR ERHÖHUNG DER BIOVERFÜGBARKEIT VON IN GEMÜSEN ODER FRÜCHTEN ENTHALTENDEN VERBINDUNGEN
PRODUIT COMESTIBLE ET SON UTILISATION POUR AUGMENTER LA BIODISPONIBILITE DE MICRONUTRIMENTS PRESENTS DANS DES LEGUMES OU DES FRUITS

(30) Priority: 01.04.2010 WO PCT/CN2010/000421
(43) Date of publication of application: 06.03.2013
(73) Proprietor: Unilever N.V., 3013 AL Rotterdam (NL); Unilever PLC, London, Greater London EC4P 0DY (GB)
(72) Inventor: BOUWENS, Elisabeth, Cornelia, Maria, NL-3133 AT Vlaardingen (NL); CAO, Xiuzhen, Shanghai 200335 (CN); FLENDRIG, Leonardus, Marcus, NL-3133 AT Vlaardingen (NL); VAN DER HIJDEN, Hendrikus, Theodorus, Wilhelmus M., NL-3133 AT Vlaardingen (NL); HU, Yingchun, Shanghai 200335 (CN)
(74) Representative: van Benthum, Wilhelmus A. J.
(86) International application number: PCT/EP2011/054691
(87) International publication number: WO 2011/120898

(56) References cited:
- WO-A1-2008/011086
- WO-A1-2008/155590
- JP-A- 2008 201 763
- JP-A- 2008 259 460
- US-A1- 2007 020 744
- US-A1- 2010 272 856
- BROWN ET AL.: "Carotenoid bioavailability is higher from salads ingested with full-fat than with fat-reduced salad dressing as measured with electrochemical detection", AM J CLIN NUTR, vol. 80, 2004, pages 396-403, XP002640990, cited in the application

## Description

### TECHNICAL FIELD OF THE INVENTION

The present invention provides an oil and enzyme containing edible product for increasing the bioavailability of one or more micronutrients that are comprised in vegetables or fruit.

### BACKGROUND OF THE INVENTION

It is well-known that fruit and vegetables are an important source of micronutrients such as vitamins and minerals. It is less well-known, however, that following consumption of fresh vegetables or fresh fruit the human body is often capable of utilizing only a fraction of the micronutrients contained therein. This phenomenon is commonly referred to as the limited oral bioavailability of micronutrients that are contained in a particular fresh vegetable or fresh fruit. Here the term "oral bioavailability" refers to the fraction of the total amount of an ingested micronutrient that ultimately reaches the systemic circulation. Thus, an oral bioavailability of 20% means that only 20% of the ingested amount of micronutrient reaches the systemic circulation.

The oral bioavailability of a micronutrient that is contained within fresh vegetables of fresh fruit is determined by three successive processes. First the micronutrient must be released from the vegetable or fruit matrix into the juices of the gastrointestinal tract. The extent to which a micronutrient is released from a vegetable or fruit into the gastrointestinal tract determines its so called bioaccessibility. Secondly, the released nutrient must enter the intestinal epithelium where it is incorporated in chylomicrons which are transported via the lymph into the bloodstream near the heart (absorption). Finally, in order to reach the systemic circulation the micronutrient that has been transported into the portal vein must pass the liver without being metabolized. Consequently, oral bioavailability of a micronutrient is determined by its bioaccessibility, its absorption and its metabolism.

In case of fresh vegetables and fresh fruit limited bioavailability of micronutrients is mainly associated with limited bioaccessibility, especially in case the micronutrient is an oil-soluble micronutrient such as an oil-soluble vitamin or provitamin. Hence, in order to effectively enhance the oral bioavailability of micronutrients in fresh vegetables and fresh fruit, the bioaccessibility of these micronutrients should be increased.

Brown et al. (Carotenoid bioavailability is higher from salads ingested with full-fat than with fat-reduced salad dressing as measured with electrochemical detection, Am J Clin Nutr (2004); 80, 396-403) have reported that the results of a study that aimed to quantify the appearance of carotenoids in plasma chylomicrons after subjects ingested fresh vegetable salads with fat-free, reduced-fat and full-fat salad dressings. The authors found that after ingestion of salads with fat-free salad the appearance of carotenoids in plasma chylomicrons was negligible. After ingestion of the salads with reduced-fat salad dressing, the appearance of the carotenoids in plasma chylomicrons increased relative to that after ingestion of the salads with fat-free salad dressing. Similarly, the appearance of the carotenoids in plasma chylomicrons was higher after the ingestion of salads with full-fat than with reduced-fat salad dressings. However, the beneficial effect on carotenoid bioavailability that can be achieved with full-fat dressings is at least to some extent outweighed by the undesirably high caloric content of such dressings. In addition, the increase in oral bioavailability that can be realized by these conventional full-fat dressings is limited, probably because these dressings have essentially no impact on the bioaccessibility of oil soluble micronutrients contained in the fresh vegetable or the fresh fruit.

Hence, there is a need for alternative means for increasing the bioavailability of nutrients that are contained in fresh vegetables and fresh fruit.

### SUMMARY OF THE INVENTION

The present inventors have succeeded in finding a new way to increase the bioavailability of nutrients that are contained in vegetables and fruit. The inventors have developed an oil containing edible product that, when consumed within 30 minutes of consuming a vegetable or a fruit containing one or more micronutrients, substantially increases the bioavailability of said micronutrients. The edible product of the present invention is selected from dressings, mayonnaise, sauces and desserts and is characterized in that it comprises 13-99 wt.% of an aqueous phase having a pH in the range of 2.0-5.5; 0.1-90 wt.% of an oil phase; 0-40 wt. of solid or semi-solid particles; and active plant cell wall degrading enzyme selected from pectinase, cellulase and combinations thereof.

JP 2000/157169 describes an oil-in-water emulsion for use in bread making, comprising 10-80 wt.% oil, water, 5-30 wt.% vinegar, 3-20 wt.% egg component, a glycolytic enzyme and optionally 1-3 wt.% stabilizer, seasoning, spice or the like.

The inventors have found that if the present edible product is consumed at about the same time as vegetables or fruit, the active cell wall degrading enzyme is capable of increasing the bioavailability of the micronutrients present in said vegetables or fruit. This increase in bioavailability results from the interaction between the enzyme and the salad components after the salad has been ingested. The oil phase of the edible product further enhances the bioavailability enhancing effect of the cell wall degrading enzyme.

Although the inventors do not wish to be bound by theory, it is believed that the favorable effect of the cell wall degrading enzyme on the bioavailability of nutrients contained in fresh vegetables and/or fresh fruit is largely attributable to the impact of the enzyme on the bioaccessibility of said micronutrients. By degrading components that are contained in the cell walls of the vegetable or fruit components whilst these components are passing through the gastrointestinal tract, the enzyme is believed to contribute to the release of micronutrients that would otherwise remain entrapped in these components until they reach a part of said gastrointestinal tract where they can no longer be absorbed. The oil components of the oil phase facilitate absorption of the micronutrients that are released a result of the activity of the cell wall degrading enzyme. Thus, because the present edible product enhances both bioaccessibility and absorption of micronutrients contained in vegetables or fruit, the edible product is surprisingly effective in raising the bioavailability of said micronutrients.

The present invention also relates to the use of an edible product for increasing the bioavailability of one or more micronutrients that are comprised in a vegetable or a fruit, wherein said edible product comprises 10-99 wt.% of an aqueous phase; 0.1-90 wt.% of an oil phase; 0-40 wt.% of solid or semi-solid particles; and active plant cell wall degrading enzyme; and wherein said use comprises consuming the edible product within 30 minutes of consuming said vegetable or fruit.

Furthermore, the inventicn provides a salad ccmprising at least 50 wt.% of vegetable and/or fruit components and at least 3 wt.% of an edible product comprising 10-99 wt.% of an aqueous phase; 0.1-90 wt.% cf an oil phase; 0-40 wt.% of solid or semi-solid particles; and active plant cell wall degrading enzyme.

### DETAILED DESCRIPTION OF THE INVENTION

Accordingly, one aspect cf the invention relates to an edible product selected from dressings, mayonnaise, sauces and desserts, and comprising 10-99 wt.% cf an aqueous phase having a pH in the range of 2.0-5.5; 0.1-90 wt.% of an oil phase; 0-40 wt.% of solid or semi-solid particles; and active plant cell wall degrading enzyme selected from pectinase, cellulase and combinations thereof.

The term "oil" as used herein refers to a lipid material that can be liquid or solid at ambient temperature. Throughout this document, unless indicated otherwise, the terms "oil" and "fat" are used interchangeable.

Unless indicated otherwise, the term "bioavailability" as user herein should be construed as "oral bioavailability".

The term "consuming" as used herein refers to the ingestion of a material, e.g. the oil containing edible product, the vegetable or the fruit.

The edible product is selected from the group consisting of dressings, mayonnaise, sauces and desserts. Preferably, the edible product is a dressing or a mayonnaise. Most preferably, the edible product is a dressing, especially a salad dressing. The aqueous phase of the edible product of the present invention has been acidified to a pH in the range of 2.0-5.5, more preferably of 2.5.-5.0. Food grade acids that may suitably be applied in the edible product include acetic acid, citric acid, lactic acid, sorbic acid, phosphoric acid, malic acid, benzoic acid, tartaric acid, ascorbic acid and combinations thereof. Most preferably, the acid is selected from acetic acid, citric acid and combinations thereof, According to a particularly preferred embodiment, the edible product contains at least 0.1 g/l of a food-grade acid as described herein before.

According to yet another advantageous embodiment of the present invention, the aqueous phase of the dressing contains 0.01- 10 wt.%, more preferably 0.05-5 wt.% of protein. Examples of proteins and protein sources that may suitably be incorporated in the aqueous phase include milk protein, egg protein, rice protein, corn protein, soy protein, mustard flour and combinations thereof. The inclusion of protein not only has a favorable impact on the taste and texture of the dressing but is also believed to further boost the advantageous bioavailability enhancing effect of the dressing.

The edible product of the present invention may suitably be combined with the vegetable or the fruit prior to consumption, e.g. by pouring it over said vegetable or fruit. In order to ensure that the product will cling to the vegetable or fruit, it is advantageous to incorporate one or more viscosifiers. Advantageously, the edible product of the present invention has a viscosity of at least 200 mPa.s, said viscosity being determined by Brookfield, spindle RV3, 30 rpm, at 20°C. Even more preferably, the edible product has a viscosity of at least 350 mPa.s.

In accordance with a particularly preferred embodiment of the present invention the edible product comprises 0.5-90 wt.% of an oil phase and 10-99 wt.% of an aqueous phase. Even more preferably, the edible product comprises 1-90 wt.% of an oil phase and 10-99 wt.% of an aqueous phase. The benefits of the present invention are particularly appreciated in case the edible product comprises 5-85 wt.%, especially 10-50 wt.% of an oil phase. Despite the relatively low concentration of oil phase, these edible products can achieve a significant increase in oral bioavailability of the oil soluble micronutrients present in a vegetable or a fruit that is consumed at about the same time as the edible product. Thus, the favorable bioavailability effect of such low caloric products can be equal or even exceed the effect observed for conventional products having much higher oil content.

The oil phase of the present edible product preferably contains at least 80 wt.%, more preferably at least 90 wt.% of glycerides selected from the group of triglycerides, diglycerides, monoglycerides, phospholipids and combinations thereof.

Typically, in the edible products of the present invention the oil phase and the aqueous phase together represent at least 70 wt.% of the product and the product may further comprise 0-30 wt.% of solid particles, e.g herbs, spices, vegetable or fruit. In order to protect the latter particles against premature enzymatic digestion, they may be coated with a protective layer (e.g. fat or wax).

In accordance with one advantageous embodiment, the edible product of the present invention is an oil-in-water emulsion. According to an alternative embodiment, the edible product is non-emulsified and comprises a layer of aqueous phase and a layer of oil phase, e.g. a dressing comprising an aqueous layer and an oil layer resting on the aqueous layer. The latter type of dressing is usually shaken to produce an emulsion that can be applied onto a salad.

The plant cell wall degrading enzyme in the present edible productpreferably is a pectinase as pectinases were found to be particularly effective. Examples of pectinases that are particularly suitable for use in the present edible product include polygalacturonase (EC 3.2.1.15) and pectin lyases (EC 4.2.2.10). Naturally, also combinations of these enzymes may be used. According to a particularly preferred embodiment, the pectinase employed in the edible product is polygalacturonase, most preferably endo-polygalacturonase.

It will be understood that the edible product of the present invention should contain a sufficient amount of the active cell wall degrading enzyme activity to achieve a substantial increase in bioavailability of micronutrients contained in a vegetable or a fruit. According to an especially preferred embodiment, the edible product exhibits sufficient pectinase activity to cause a viscosity decrease at 20°C and a shear rate of 1 s⁻¹ of at least 20% when 30 µl of the product is mixed with 2 ml of a 3.5% (w/w) pectin solution, using the test procedure described in the Examples. More preferably, the edible product exhibits sufficient pectinase activity to cause a viscosity decrease of at least 40%, even more preferably of at least 50% and most preferably of at least 60% in the aforementioned assay

The cellulase that can be employed in the present edible product is suitably selected from endo-cellulase, exo-cellulase and combinations thereof. Preferably, the edible product exhibits sufficient cellulase activity to cause a viscosity decrease at 20°C and a shear rate of 1 s⁻¹ of at least 20% when 30 µl of the product is mixed with 2 ml of a 1.5% (w/w) carboxymethyl cellulose solution, using the test procedure described in the Examples. More preferably, the edible product exhibits sufficient cellulase activity to cause a viscosity decrease of at least 40%, even more preferably of at least 50% and most preferably of at least 60% in the aforementioned assay.

The inventors have found that surprisingly good results can be obtained if the edible product exhibits both significant pectinase and cellulase activity. Hence, in accordance with a particularly preferred embodiment, the edible product exhibits sufficient pectinase activity to cause a viscosity decrease of at least 20% in the aforementioned pectinase assay and, in addition, the edible product exhibits sufficient cellulase activity to cause a viscosity decrease of at least 20% in the above mentioned cellulase assay.

It is preferred that the edible product contains only a limited amount of substrate that can be digested by the cell wall degrading enzyme. Accordingly, in a preferred embodiment the edible product contains less than 0.5 wt.%, more preferably less than 0.1 wt.% and most preferably less than 0.03 wt.% of pectin that can be digested *in situ* by the cell wall degrading enzyme. Likewise, the edible product preferably contains less than 0.5 wt.%, more preferably less than 0.1 wt.%, and most preferably less than 0.03 wt.% of cellulose that can be digested *in situ* by the cell wall degrading enzyme.

Another aspect of the invention relates to an edible product as defined herein before, wherein the edible product is packaged in a sealed container, said container carrying one or more statements indicating that the consumption of the edible product increases the bioavailability of micronutrients that are comprised in vegetables or fruit.

In accordance with a particularly preferred embodiment, the edible product is a salad dressing and the one or more statements on the container indicate that the salad dressing can be used on a salad to increase the bioavailability of micronutriens that are comprised in the vegetable or fruit components of the salad.

A further aspect of the invention relates to a salad comprising at least 50 wt.% of vegetable and/or fruit components and at least 3 wt.% of an edible product comprising 10-99 wt.% of an aqueous phase; 0.1-90 wt.% of an oil phase; 0-40 wt.% of solid or semi-solid particles; and active plant cell wall degrading enzyme. The cell wall degrading enzyme is advantageously selected from pectinase, cellulase, hemicellulase and combinations thereof.

According to a particularly preferred embodiment, the salad comprises an edible product as defined herein before. Particularly preferred is a salad that comprises at least 40 wt.%, preferably at least 70 wt.% of one or more vegetables selected from lettuce, tomato, carrot, beetroot and combinations thereof.

Yet another aspect of the present invention relates to the use of an edible product for increasing the bioavailability of one or more micronutrients that are comprised in a vegetable or a fruit, wherein said edible product comprises 10-99 wt.% of an aqueous phase; 0.1-90 wt.% of an oil phase; 0-40 wt.% of solid or semi-solid particles; and active plant cell wall degrading enzyme; and wherein said use comprises consuming the edible product within 30 minutes of consuming said vegetable or fruit. The cell wall degrading enzyme is advantageously selected from pectinase, cellulase, hemicellulase and combinations thereof.

According to a particularly preferred embodiment, the edible product that is used for increasing the bioavailability of one or more micronutrients is an edible product as defined herein before.

According to one embodiment of the above mentioned use, the edible product and the vegetable or fruit are consumed separately, i.e. ingested separately, but the time period between these separate consumption events does not exceed 30 minutes. Here the time period between these consumption events is defined as the time period that starts with the end of the first event and that ends with the start of the other event. According to a particularly preferred embodiment, the edible product is consumed within 20 minutes, most preferably within 10 minutes of consuming the vegetable or the fruit. It should be understood that in accordance with this embodiment, the edible product can be consumed prior or after consumption of the vegetable or the fruit.

According to another, particularly preferred embodiment, the edible product is combined with the vegetable or the fruit prior to consumption. Examples of this particular embodiment include the combination of a salad and a salad dressing, the combination of cooked vegetables and a sauce, and the combination of fruit and a dessert.

The edible product of the present invention can suitably be used to enhance the bioavailability of a wide range of micronutrients, especially oil soluble micronutrients. Examples of such oil-soluble micronutrients include provitamin A, vitamin E, vitamin K and lycopene. Examples of provitamin A whose bioavailability can be enhanced effectively by using the present edible product include a-carotene, β-carotene, γ-carotene and β-cryptoxanthin.

The fruit or vegetable containing the micronutrients can be fresh or cooked. According to a particularly preferred embodiment, the vegetable or fruit is a fresh vegetable or a fresh fruit.

In accordance with a particularly preferred embodiment of the present use, the edible product is a salad dressing and the vegetable or fruit is a component of a salad. Advantageously, said salad dressing is combined with the salad prior to consumption. Preferably, the salad comprises at least 40 wt.%, more preferably at least 70 wt.% of one or more fresh vegetables selected from lettuce, tomato, carrot, beetroot and combinations thereof.

In accordance with the present use, the salad dressing can be combined with the salad in numerous ways. A convenient way of combining the dressing with the salad is to pour the salad dressing over a freshly prepared salad.

The invention is further illustrated by means of the following, non-limiting examples.

### EXAMPLES

### Assay for determining pectinase activity:

A solution containing 3.5 wt.% of low methoxyl pectin and having a pH of 3.0 is prepared as follows:
- Add 3.5 g LM citrus pectin (Grindsted® pectin LC 950 from Danisco) and citric acid (0.19 g/l) to 96 g demineralised water under continuous mixing and then add 0.876g NaCl;
- Mix the solution for at least 30 min;
- Heat the solution for 5 min at 100 °C.

Pectinase activity is measured using the following procedure:
- Take at least two samples from the product to be tested;
- Prepare a control sample by heating it to 10 minutes at 95 °C to destroy pectinase activity;
- Add 30 ul of each sample to 2 ml of the LM pectin solution and transfer them into an Eppendorf;
- Turn the Eppendorf upside down 3x;
- Incubate the samples 15 min at 37 °C using an Eppendorf thermostatic shaker at 1000 rpm;
- Heat the samples for 10 min 95°C;
- Measure the difference in viscosity between the test sample and the control sample using a TA AR 1000 rheometer (cone plate diameter 4 cm, gap 71 micron, angle 1.59°). Method: flow procedure; parameters: peak hold; hold at 1 s⁻¹ shear, duration 1 min (20 data points); equilibration 1 min, at 20 °C.

Activity is given as % decrease in viscosity of the pectin solution compared with a standard dressing (sample without enzyme activity).

### Assay for determining cellulase activity:

Procedure is identical to the procedure described above for the pectinase assay, except that instead of solution containing 3.5 wt.% of LM pectin, a solution containing 1.5 wt.% of carboxymethyl cellulose is employed.

### Example 1

Dressings were prepared on the basis of the formulation depicted in Table 1.

**Table 1**

| **Ingredient** | **Wt%** |
|---|---|
| Aqueous solution of salt and sucrose | 53.513 |
| Vinegar White | 17.400 |
| Vegetable oil | 21.300 |
| Herbs & spices | 3.500 |
| Flavouring & colouring | 0.800 |
| Xanthan gum | 0.180 |
| Calcium Disodium EDTA | 0.007 |
| Enzyme preparation | 3.300 |
| **Total** | **100** |

The following commercially available enzyme preparation were included in the dressings:
A. Depol™ 670L (Biocatalysts Ltd., Wales, UK)
B. Glucanase 5XL (Biocatalysts Ltd., Wales, UK)
C. Depol™ 40L (Biocatalysts Ltd., Wales, UK)
D. Cellulase C013L (Biocatalysts Ltd., Wales, UK)
E. Ultrazyme™AFP L (Novozymes A/S, Bagsvaerd, Denmark)
F. Viscozyme™ L (Novozymes A/S, Bagsvaerd, Denmark)
G. Rapidase® FC (DSM Food Specialties B.V, Delft, the Netherlands)
H. Klerzyme® 150 (DSM Food Specialties B.V, Delft, the Netherlands)
I. Pectinase 62L (Biocatalysts Ltd., Wales, UK)

All of the dressings so prepared exhibited a pectinase activity of at least 60% in the pectinase assay described herein before. Most of the dressings further exhibited a cellulase activity of at least 60% in the cellulase assay described herein before.

### Example 2

The effect of the different enzyme preparations on the bioaccessibility of lipophilic micronutrients contained in a salad was assessed in an *in vitro* bioaccessibility test. To this end, model dressings were prepared in accordance with the recipe described in Table 2.

**Table 2**

| **Ingredient** | Control (wt%) | Enzyme (wt%) |
|---|---|---|
| Aqueous solution of salt and sucrose | 98.13 | 95.63 |
| Acetic acid (100%) | 0.62 | 0.62 |
| Lecithin ^{#} | 1.25 | 1.25 |
| Enzyme preparation | - | 2.5 |

| | | |
|---|---|---|
| ^{#} Epikuron™ 200, Cargill, Inc. | | |

These model dressing were subjected to an *in vitro* bioaccessibility test using the following protocol.

Salad samples were prepared by adding 200 grams Romaine Lettuce + 200 grams of water in a blender and processing them until salad particles were obtained which were comparable to the size after normal chewing. 50 grams of this salad mixture (comprising 25 grams salad) was then put into the reaction vessel of the in-vitro digestion model. Onto this, 10 grams of the salad dressing was added by separately pipetting the aqueous phase, the oil phase, and the enzyme preparation of the dressing. In case of the control, enzyme was replaced by water.

Effects on bioaccessibility were evaluated by determining the nutrient content of the vegetable and by measuring the in vitro extraction of micronutrients in digestive juices. Artificial digestive juices were prepared based on human physiology and added to the products, mixed and incubated according to physiological pH and transit times. The fraction of micronutrients that is released from the salad components into the aqueous volume (dissolved or solubilised in mixed micelles) of the digestive juices after centrifugation and filtration represents the bioaccessible fraction. The relative *in vitro* accessibility of a micronutrient can be calculated as the *in vitro* accessible amount over the total amount of that nutrient present in the vegetable and expressed as a percentage.

The results of the study are summarized in Table 3 (β-carotene), Table 4 (lutein) and Table 5 (folate).

**Table 3**

| **β-carotene** | % **Bioacessibility (Average ± SD)** | |
|---|---|---|
| | **Control** | **Invention** |
| Depol™ 670L | 48.2 ± 9.1 | 111.8 ± 9.0 |
| Glucanase 5XL | 56.1 ± 1.6 | 105.3 ± 7.7 |
| Depol™ 40L | 48.2 ± 9.1 | 98.8 ± 13.3 |
| Cellulase C013L | 57.1 ± 7.8 | 87.5 ± 7.1 |
| Viscozyme™ L | 53.8 ± 0.2 | 93.6 ± 2.9 |
| Ultrazyme™ AFP L | 57.1 ± 7.8 | 84.9 ± 0.3 |
| Klerzyme® 150 | 61.4 ± 5.2 | 106.0 ± 5.4 |
| Rapidase® FC | 52.7 ± 2.4 | 104.9 ± 6.1 |

**Table 4**

| **Lutein** | **% Bioacessibility (Average ± SD)** | |
|---|---|---|
| | **Control** | **Invention** |
| Depol™ 670L | 56.1 ± 5.6 | 91.8 ± 1.5 |
| Glucanase 5XL | 54.3 ± 2.9 | 95.6 ± 9.8 |
| Depol™ 40L | 56.1 ± 5.6 | 88.7 ± 10.7 |
| Cellulase C013L | 56.2 ± 4.0 | 87.2 ± 3.4 |
| Viscozyme™ L | 53.4 ± 1.5 | 83.5 ± 3.4 |
| Ultrazyme™ AFP L | 56.2 ± 2.9 | 83.2 ± 3.2 |
| Pectinase 62L | 56.2 ± 4.0 | 80.6 ± 1.7 |

**Table 5**

| **Folate** | **% Bioacessibility (Average ± SD)** | |
|---|---|---|
| | **Control** | **Invention** |
| Cellulase C013L | 37.2 ± 1.2 | 90.6 ± 23.3 |

These results clearly demonstrate that edible products containing active cell wall degrading enzymes having pectinase and/or cellulase activity are capable of substantially enhancing the bioaccessibility of micronutrients that are contained in fruit or vegetables if these edible products are consumed together with such fruit or vegetables.

### Example 3

The effect of pectinase and cellulase activity on the bioaccessibility of lipophilic micronutrients contained in a salad was assessed using the *in vitro* bioaccessibility test described in Example 2. This time, instead of commercial enzyme preparations that typically exhibit a wide spectrum of enzyme activities, highly purified enzyme preparations were used, i.e. a pure endo-polygalacturonase (Polygalacturonase M2 (*A. aculeatus*), supplied by Megazyme) and a pure cellulase (Carezyme 1000L®; C2605 Cellulase from *Aspergillus sp.,* supplied by Sigma).

The compositions of the model dressings employed in the bioaccessibility test are described in Table 6.

**Table 6**

| **Ingredient** | Dressing I | Dressing II | Dressing III |
|---|---|---|---|
| Aqueous solution of salt and sucrose | 95.63 wt.% | 93.13 wt.% | 90.63 wt.% |
| Acetic acid (100%) | 0.62 wt.% | 0.62 wt.% | 0.62 wt.% |
| Lecithin ^{#} | 1.25 wt.% | 1.25 wt.% | 1.25 wt.% |
| Polygalacturonase | 2.5 wt.% | | 2.5 wt.% |
| Cellulase | | 5.0 wt.% | 5.0 wt.% |

The results obtained from the bioaccessibility test (increased in bioavailability) are depicted in Table 7.

**Table 7**

| **β-carotene** | Bioaccessibility (%) ± SD |
|---|---|
| Dressing I | 37.6 ± 0.3 |
| Dressing II | 48.8 ± 3.8 |
| Dressing III | 72.5 ± 7.1 |

These results show that the combined action of polygalacturonase and cellulase produces a surprisingly big increase in bioaccessibility of lipophilic micronutrients.

### Example 4

The effect of cell wall degrading enzymatic activity on the bioaccessibility of vitamin C contained in a salad was assessed using the *in vitro* bioaccessibility test described in Example 2.

The compositions of the model dressings employed in the bioaccessibility test are described in Table 8.

**Table 8**

| **Ingredient** | Control (wt%) | Enzyme (wt%) |
|---|---|---|
| Aqueous solution of salt and sucrose | 98.13 | 95.63 |
| Acetic acid (100%) | 0.62 | 0.62 |
| Lecithin ^{#} | 1.25 | 1.25 |
| Enzyme preparation | - | 2.5 |

| | | |
|---|---|---|
| ^{#} Epikuron™ 200, Cargill, Inc. | | |

The results obtained from the bioaccessibility test after digestion of salad with the respective salad dressings are depicted in Table 9.

**Table 9**

| | Bio accessible Ascorbic acid (mg/l) | % increase compared with reference |
|---|---|---|
| 1) Reference | 75 | 0 |
| 2) Cytolase | 103 | 37 |
| 3) Viscozyme | 103 | 37 |
| 4) Ultrazyme | 105 | 40 |
| 5) Depol 392 | 89 | 19 |
| 6) Rapidase TF | 90 | 20 |
| 7) Rapidase FC | 88 | 17 |
| 8) Klerzyme | 103 | 37 |

| | | |
|---|---|---|
| # Ascorbic acid concentration was measured using Reflectoquant™ strips from Merck, Germany | | |

## Claims

1. An edible product selected from dressings, mayonnaise, sauces and desserts, said edible product comprising 10-99 wt.% of an aqueous phase having a pH in the range of 2.0-5.5; 0.1-90 wt.% of an oil phase; 0-40 wt.% of solid or semi-solid particles; and active plant cell wall degrading enzyme selected from pectinase, cellulase and combinations thereof.

2. Edible product according to claim 1, wherein the aqueous phase has a pH in the range of 2.5-5.0.

3. Edible product according to claim 1 or 2, wherein the aqueous phase contains at least 0.1 g/l of food-grade acid selected from acetic acid, citric acid, lactic acid, sorbic acid, phosphoric acid, ascorbic acid and combinations thereof.

4. Edible product according to any one of the preceding claims, wherein the product contains 1-90 wt.% of an oil phase.

5. Edible product according to any one of the preceding claims, wherein the oil phase and the aqueous phase together represent at least 70 wt.% of the product and wherein the product comprises up to 30 wt.% of solid or semi-solid particles.

6. Edible product according to any one of the preceding claims, wherein the product has a viscosity of at least 200 mPa.s, said viscosity being determined by Brookfield, spindle RV3, 30 rpm, at 20°C.

7. Edible product according to any one of the preceding claims, wherein the active plant cell wall degrading enzyme is pectinase.

8. Edible product according to claim. 7, wherein the pectinase is selected from polygalacturonase (EC 3.2.1.15) and pectin lyases (EC 4.2.2.10).

9. Edible product according to claim 8, wherein the pectinase is polygalacturonase, preferably endo-polygalacturonase.

10. A salad comprising at least 50 wt.% of vegetable and/or fruit components and at least 3 wt.% of an edible product comprising 10-99 wt.% of an aqueous phase; 0.1-90 wt.% of an oil phase; 0-40 wt.% of solid or semi-solid particles; and active plant cell wall degrading enzyme.

11. Salad according to claim 10, wherein the edible product is an edible product according to any one of claims 1-11.

12. Salad according to claim 10 or 11, wherein the salad comprises at least 40 wt.%, preferably at least 70 wt.% of one or more vegetables selected from lettuce, tomato, carrot, beetroot and combinations thereof.

13. Use of an edible product for increasing the bioavailability of one or more micronutrients that are comprised in a vegetable or a fruit, wherein said edible product comprises 10-99 wt.% of an aqueous phase; 0.1-90 wt.% of an oil phase; 0-40 wt.% of solid or semi-solid particles; and active plant cell wall degrading enzyme; and wherein said use comprises consuming the edible product within 30 minutes of consuming said vegetable or fruit:

14. Use according to claim 13, wherein the edible product is an edible product according to any one of claims 1-11.

15. Use according to claim 14, wherein the edible product is a salad dressing and wherein said salad dressing is combined with a salad containing the vegetable or the fruit prior to consumption.

## Patentansprüche

1. Essbares Produkt, ausgewählt aus Dressings, Mayonnaise, Soßen und Desserts, wobei das essbare Produkt Folgendes umfasst: 10-99 Gew.-% einer wässrigen Phase mit eine pH-Wert im Bereich von 2,0-5,5; 0,1-90 Gew.-% einer Ölphase; 0-40 Gew.-% feste oder halbfeste Teilchen; und ein aktives, Pflanzenzellwände abbauendes Enzym, das aus Pectinase, Cellulase und Kombinationen davon ausgewählt ist.

2. Essbares Produkt nach Anspruch 1, wobei die wässrige Phase einen pH-Wert im Bereich von 2,5-5,0 aufweist.

3. Essbares Produkt nach Anspruch 1 oder 2, wobei die wässrige Phase mindestens 0,1 g/Liter einer Säure für Nahrungsmittelzwecke umfasst, die aus Essigsäure, Citronensäure, Milchsäure, Sorbinsäure, Phosphorsäure, Ascorbinsäure und Kombinationen davon ausgewählt ist.

4. Essbares Produkt nach einem der vorstehenden Ansprüche, wobei das Produkt 1-90 Gew.-% einer Ölphase enthält.

5. Essbares Produkt nach einem der vorstehenden Ansprüche, wobei die Ölphase und die wässrige Phase zusammen mindestens 70 Gew.-% des Produkts ausmachen und das Produkt bis zu 30 Gew.-% feste oder halbfeste Teilchen umfasst.

6. Essbares Produkt nach einem der vorstehenden Ansprüche, wobei das Produkt eine Viskosität von mindestens 200 mPa-s aufweist, wobei die Viskosität gemäß Brookfield, Spindel RV3, 30 U/min, bei 20 °C bestimmt wird.

7. Essbares Produkt nach einem der vorstehenden Ansprüche, wobei es sich beim aktiven, Pflanzenzellwände abbauenden Enzym um Pectinase handelt.

8. Essbares Produkt nach Anspruch 7, wobei die Pectinase ausgewählt ist aus Polygalacturonase (EC 3.2.1.15) und Pectin-lyasen (EC 4.2.2.10).

9. Essbares Produkt nach Anspruch 8, wobei es sich bei der Pectinase um Polygalacturonase und vorzugsweise um Endo-polygalcturonase handelt.

10. Salat, umfassend mindestens 50 Gew.-% Gemüse- und/oder Fruchtkomponenten und mindestens 3 Gew.-% eines essbaren Produkts, das 10-99 Gew.-% einer wässrigen Phase; 0,1-90 Gew.-% einer Ölphase; 0-40 Gew.-% feste oder halbfeste Teilchen, und ein aktives, Pflanzenzellwände abbauendes Enzym umfasst.

11. Salat nach Anspruch 10, wobei es sich beim essbaren Produkt um ein essbares Produkt nach einem der Ansprüche 1 bis 11 handelt.

12. Salat nach Anspruch 10 oder 11, wobei der Salat mindestens 40 Gew.-% und vorzugsweise mindestens 70 Gew.-% einer oder mehrere Gemüsearten umfasst, die aus Lattich, Tomaten, Karotten, roten Beten und Kombinationen davon ausgewählt sind.

13. Verwendung eines essbaren Produkts zur Erhöhung der biologischen Verfügbarkeit von einem oder mehreren Mikronährstoffen, die in einem Gemüse oder einer Frucht enthalten sind, wobei das essbare Produkt 10-99 Gew.-% einer wässrigen Phase; 0,1-90 Gew.-% einer Ölphase; 0-40 Gew.-% feste oder halbfeste Teilchen, und ein aktives, Pflanzenzellwände abbauendes Enzym umfasst, wobei die Verwendung den Verzehr des essbaren Produkts innerhalb von 30 Minuten vor oder nach Verzehr des Gemüses oder der Frucht umfasst.

14. Verwendung nach Anspruch 13, wobei es sich beim essbaren Produkt um ein essbares Produkt nach einem der Ansprüche 1 bis 11 handelt.

15. Verwendung nach Anspruch 14, wobei es sich beim essbaren Produkt um ein Salatdressing handelt und wobei das Salatdressing mit einem Salat, der das Gemüse oder die Frucht enthält, vor dem Verzehr vereinigt wird.

## Revendications

1. Produit comestible sélectionné parmi des assaisonnements, de la mayonnaise, des sauces et des desserts, ledit produit comestible comprenant 10-99 % en poids d'une phase aqueuse ayant un pH dans la plage de 2,0-5,5 ; 0,1-90 % en poids d'une phase huileuse ; 0-40 % en poids de particules solides ou semi-solides ; et une enzyme de dégradation des parois des cellules végétales active sélectionnée parmi la pectinase, la cellulase et des combinaisons de celles-ci.

2. Produit comestible selon la revendication 1, dans lequel la phase aqueuse possède un pH dans la plage de 2,5 à 5,0.

3. Produit comestible selon la revendication 1 ou 2, dans lequel la phase aqueuse contient au moins 0,1 g/l d'un acide de qualité alimentaire sélectionné parmi l'acide acétique, l'acide citrique, l'acide lactique, l'acide sorbique, l'acide phosphorique, l'acide ascorbique et des combinaisons de ceux-ci.

4. Produit comestible selon l'une quelconque des revendications précédentes, où le produit contient 1 à 90 % en poids d'une phase huileuse.

5. Produit comestible selon l'une quelconque des revendications précédentes, dans lequel la phase huileuse et la phase aqueuse représentent ensemble au moins 70 % en poids du produit et où le produit comprend jusqu'à 30 % en poids de particules solides ou semi-solides.

6. Produit comestible selon l'une quelconque des revendications précédentes, où le produit possède une viscosité d'au moins 200 mPa.s, ladite viscosité étant déterminée par un viscosimètre de type Brookfield doté d'un mobile RV3, à 30 tr/min et à 20 °C.

7. Produit comestible selon l'une quelconque des revendications précédentes, dans lequel l'enzyme de dégradation des parois des cellules végétales active est la pectinase.

8. Produit comestible selon la revendication 7, dans lequel la pectinase est sélectionnée parmi la polygalacturonase (EC 3.2.1.15) et des pectine lyases (EC 4.2.2.10).

9. Produit comestible selon la revendication 8, dans lequel la pectinase est la polygalacturonase, de préférence l'endopolygalacturonase.

10. Salade comprenant au moins 50 % en poids de composants de légumes et/ou de fruits et au moins 3 % en poids d'un produit comestible comprenant 10 à 99 % en poids d'une phase aqueuse ; 0,1 à 90 % en poids d'une phase huileuse ; 0 à 40 % en poids de particules solides ou semi-solides ; et une enzyme de dégradation des parois des cellules végétales active.

11. Salade selon la revendication 10, dans laquelle le produit comestible est un produit comestible selon l'une quelconque des revendications 1 à 11.

12. Salade selon la revendication 10 ou 11, où la salade comprend au moins 40 % en poids, de préférence au moins 70 % en poids d'un ou de plusieurs légumes sélectionnés parmi la laitue, la tomate, la carotte, la betterave et des combinaisons de ceux-ci.

13. Utilisation d'un produit comestible pour augmenter la biodisponibilité d'un ou de plusieurs micronutriments qui sont compris dans un légume ou un fruit, où ledit produit comestible comprend 10 à 99 % en poids d'une phase aqueuse ; 0,1 à 90 % en poids d'une phase huileuse ; 0 à 40 % en poids de particules solides ou semi-solides ; et une enzyme de dégradation des parois des cellules végétales active ; et où ladite utilisation comprend la consommation du produit comestible dans un intervalle de 30 minutes par rapport à la consommation dudit légume ou fruit.

14. Utilisation selon la revendication 13, dans laquelle le produit comestible est un produit comestible selon l'une quelconque des revendications 1 à 11.

15. Utilisation selon la revendication 14, dans laquelle le produit comestible est un assaisonnement pour salade et où ledit assaisonnement pour salade est combiné avec une salade contenant le légume ou le fruit avant la consommation.
